# EUROPÄISCHE PATENTANMELDUNG

(11) **EP 0 924 190 A2**
(43) Veröffentlichungstag der Anmeldung: **23.06.1999**
(21) Anmeldenummer: 98123146.7
(22) Anmeldetag: 04.12.1998
(51) Int. Cl.: C07C 209/64

(54) **Herstellung von sekundären Diarylaminen**

(30) Priorität: 17.12.1997 DE 19756158
(71) Anmelder: Bayer Aktiengesellschaft, 51368 Leverkusen (DE)
(72) Erfinder: Baumgartner, Hanspeter Dr., 47807 Krefeld (DE); Klausener, Alexander Dr., 50259 Pulheim (DE)

(57) **Zusammenfassung**

Sekundäre Diarylamine der Formel 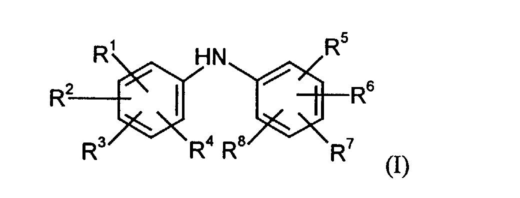 können durch diskontinuierliche oder kontinuierliche Umsetzung von primären Arylaminen der Formeln 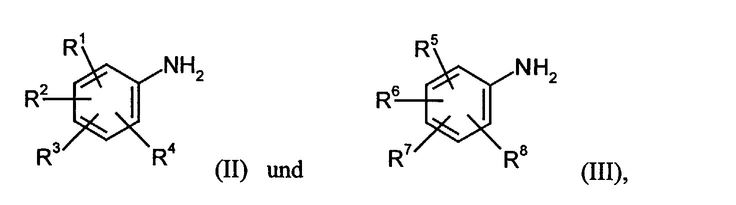 in denen R¹ bis R⁸ die in der Beschreibung angegebene Bedeutung haben, in Gegenwart eines Fluor und gegebenenfalls Bor enthaltenden Katalysators hergestellt werden. Das Verfahren wird in kondensierter Phase bei 100-400°C und gegebenenfalls unter Druck bis zu 30 bar durchgeführt.

## Beschreibung

Die vorliegende Erfindung betrifft ein Verfahren zur Herstellung von sekundären Diarylaminen durch Umsetzung von primären Arylaminen, worin die Reaktion in kondensierter Phase und in Gegenwart eines Fluor und gegebenenfalls Bor enthaltenden Katalysators durchgeführt wird, der Ammoniumfluorid und/oder Ammoniumbifluorid ist oder aus Ammoniumfluorid und/oder Ammoniumbifluorid und einer Bor enthaltenden Komponente hergestellt wird.

Sekundäre Diarylamine finden vielfache industrielle Verwendung, etwa als Ausgangsmaterialien zur Herstellung von Farbstoffen oder Stabilisatoren für Polymere oder Schmieröle. Diphenylamin ist beispielsweise eine wichtige Ausgangsverbindung zur Herstellung von Kautschukstabilisatoren des 4-N-Alkylamino-diphenylamin-Typs. Weiterhin findet Diphenylamin als stabilisierender Zusatz bei Heizölen Verwendung. N-Phenyl-1-naphthylamin wird beispielsweise als Alterungs- oder Oxidationsschutzmittel für Kautschuk sowie für Mineralölprodukte eingesetzt (Ullmanns Encyklopädie der technischen Chemie, 4. Auflage, Bd. 17, S. 107, Verlag Chemie, Weinheim 1979). Weitere Einsatzmöglichkeiten finden sich auf dem Gebiet der Farbstoffe. So erhält man etwa den Farbstoff Victoriablau, ein Diphenyl-naphthylmethan-Derivat, ausgehend von N-Phenyl-1-naphthylamin (H. E. Fierz-David und L. Blangley, Farbenchemie, 8. Aufl., S. 171, Springer-Verlag, Wien 1952). Auch Derivate des Diphenylamins oder des N-Phenyl-1-naphthylamins, die entweder aus diesen Stoffen selbst oder auf analoge Weise zu diesen Stoffen hergestellt werden, sind als Komponenten von Schmiermitteln von Interesse (EP 716 141).

Nach DE-OS 2 521 293 wird Diphenylamin durch die Umsetzung von Anilin unter erhöhtem Druck und in Gegenwart eines aus wäßriger Flußsäure und Borsäure bereiteten Katalysators hergestellt. Nachteilig ist hierbei die Verwendung des stark korrosiven, schwierig zu handhabenden und unter dem Gesichtspunkt der Arbeitssicherheit problematischen Fluorwasserstoffs. Nach H. E. Fierz-David und L. Blangley, Farbenchemie, loc. cit., erhält man N-Phenyl-1-naphthylamin in Ausbeuten von 86-91 % der theoretischen Ausbeute durch Umsetzung von Anilin und 1-Naphthylamin bei Temperaturen von 195-215°C unter Normaldruck, wobei Sulfanilsäure als Katalysator dient. Hier erfolgt keine Wiedergewinnung des Katalysators, so daß die ökonomischen und ökologischen Nachteile dieselben sind. Gleiches gilt für die Reaktionszeiten. Nach einer in DE-PS 241 853 sowie im J. Prakt. Chem. 89, 1 (1914) beschriebenen Vorgehensweise erhält man N-Phenyl-1-naphthylamin durch Umsetzung von Anilin und 1-Naphthylamin bei Temperaturen von 225-250°C in Gegenwart katalytischer Mengen Jod unter Normaldruck. Auch bei dieser Variante wird der Katalysator nicht wiederverwendet, so daß sich auch hier ökonomische und ökologische Nachteile ergeben. Die beschriebenen Reaktionsausbeuten sind zudem deutlich geringer als in den o.g. Beispielen, und auch die Reaktionszeiten sind unvorteilhaft lang. Nach J. Prakt. Chem., loc. cit., kann N-Phenyl-1-naphthylamin durch Umsetzung von 1-Naphthol mit Anilin bei Temperaturen zwischen 180 und 200°C ebenfalls in Gegenwart katalytischer Mengen Jod unter Normaldruck gewonnen werden. Die Ausbeuten erreichen jedoch nur 35-40 % der theoretischen Ausbeute, was diese Variante deutlich ungünstiger als die oben beschriebenen erscheinen läßt.

Allen genannten Verfahren ist zudem gemeinsam, daß in ihrem Verlauf mehr oder weniger große Mengen unerwünschter Nebenprodukte, wie beispielsweise Diphenylamin, N-Phenyl-2-naphthylamin sowie 2-Naphthylamin, gebildet werden. Vor allem die letztgenannten Verbindungen N-Phenyl-2-naphthylamin und 2-Naphthylamin können grundsätzlich durch Isomerisierung von bereits gebildetem N-Phenyl-1-naphthylamin bzw. nicht umgesetztem 1-Naphthylamin entstehen, und zwar in Abhängigkeit von den zur Anwendung kommenden Reaktionsbedingungen und dem verwendeten Katalysator. Abgesehen davon, daß die Bildung derartiger unerwünschter Nebenprodukte aufgrund damit verbundener Materialverluste und dem erforderlich werdenden erhöhten trenntechnischen Aufwand zur Isolierung von reinem N-Phenyl-1-naphthylamin eine erhebliche Beeinträchtigung der Wirtschaftlichkeit des jeweiligen Herstellungsprozesses nach sich zieht, ist ferner zu berücksichtigen, daß insbesondere 2-Naphthylamin stark toxische Eigenschaften besitzt und seine Entstehung daher möglichst auszuschließen ist.

Es bestand somit die Aufgabe, ein wirtschaftliches, möglichst unproblematisch zu handhabende Chemikalien verwendendes Verfahren zur hochselektiven Herstellung von sekundären Diarylaminen, insbesondere der wirtschaftlich interessanten Verbindungen Diphenylamin und N-Phenyl-1-naphthylamin, zu finden, bei dem die Bildung unerwünschter Nebenprodukte weitgehend ausgeschlossen ist, das die Rückführung des zur Anwendung kommenden Katalysators erlaubt und das mit wirtschaftlich zufriedenstellenden Reaktionszeiten bzw. Raum-Zeit-Ausbeuten arbeitet.

Es wurde überraschenderweise gefunden, daß sich sekundäre aromatische Amine in hohen Ausbeuten und ausgezeichneten Selektivitäten erhalten lassen, wenn man primäre aromatische Amine der im folgenden genannten Formeln in Gegenwart eines Fluor und gegebenenfalls Bor enthaltenden Katalysators, der Ammoniumfluorid oder Ammoniumbifluorid ist oder aus Ammoniumfluorid oder Ammoniumbifluorid und einer Bor enthaltenden Komponente hergestellt wird, miteinander umsetzt. Der unerwartete Vorteil liegt dabei vor allem darin, daß es zur Herstellung des erfindungsgemäßen Katalysatorsystems nicht, wie in DE-OS 2521293 beschrieben, des hochreaktiven, aber schwierig zu handhabenden Fluorwasserstoffs oder der wäßrigen Flußsäure bedarf, und daß sich dadurch vor allem die industrielle Herstellung der genannten sekundären aromatischen Amine unter den Gesichtspunkten der Arbeitssicherheit, der Arbeitshygiene und der Wirtschaftlichkeit in deutlich verbesserter Weise durchführen läßt. Es kann vielmehr auf handelsübliche Fluoride zurückgegriffen werden. Dabei war weiterhin die Beobachtung von Interesse, daß die erfindungsgemäß zum Einsatz kommenden Katalysatoren auf einfache Weise, also ohne Reinigung und Aufarbeitung, durch wäßrige Extraktion des ausreagierten Reaktionsgemisches wiedergewonnen werden können. Die solchermaßen wiedergewonnenen Katalysatoren können in die Reaktion zurückgeführt werden, ohne daß dies zu negativen Folgen für Umsatz und Selektivität führt.

Angesichts des genannten Standes der Technik konnten diese günstigen Ergebnisse nicht erwartet werden. Vielmehr hätte man aufgrund der erheblich geringeren Reaktivität der Fluor enthaltenden Katalysatorkomponenten mit deutlich verlängerten Reaktionszeiten und mit der Bildung größerer Mengen unerwünschter Nebenprodukte rechnen müssen. Vergleicht man das erfindungsgemäße Verfahren mit denen, die aus der Literatur bekannt sind, so hätte man im übrigen einen Verlust des Katalysators nach Durchführung der Reaktion erwarten müssen.

Es wurde ein Verfahren zur Herstellung von sekundären Diarylaminen der Formel (I) 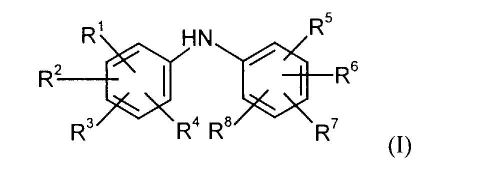 durch Umsetzung von primären Arylaminen der Formel (II) 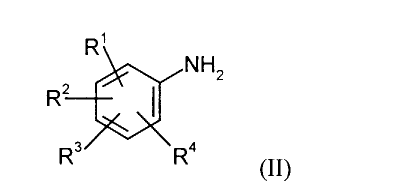 mit primären aromatischen Aminen der Formel (III) 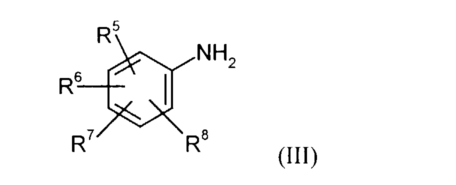 gefunden, wobei
R¹, R², R³, R⁴, R⁵, R⁶, R⁷ und R⁸ gleich oder verschieden sind und für Wasserstoff, geradkettiges oder verzweigtes C₁-C₃₀-Alkyl, C₂-C₃₀-Alkenyl, Halogen, C₁-C₈-Alkoxy, Trifluormethyl, C₁-C₈-Alkylamino, Di(C₁-C₈-alkyl)amino, Carboxyl, C₁-C₈-Alkoxycarbonyl oder Phenyl stehen, wobei jeweils zwei ortho-ständige Substituenten aus R¹, R², R³ und R⁴ und/oder aus R⁵, R⁶, R⁷ und R⁸ einen gesättigten oder einen einfach oder zweifach ungesättigten 5- oder 6-gliedrigen Carbocyclus bilden können, das dadurch gekennzeichnet ist, daß die Reaktion diskontinuierlich oder kontinuierlich in Gegenwart eines Fluor und gegebenenfalls Bor enthaltenden Katalysators aus der Gruppe von gegebenenfalls substituiertem Ammoniumfluorid, Ammoniumbifluorid und Katalysatoren, die aus gegebenenfalls substituiertem Ammoniumfluorid und/oder Ammoniumbifluorid und einer Bor enthaltenden Verbindung hergestellt werden, bei 100-400°C in kondensierter Phase durchgeführt wird, wobei bei Mitverwendung eines Bor enthaltenden Katalysators die Herstellung von N-Phenyl-1-naphtylamin ausgenommen ist.

Geradkettiges oder verzweigtes C₁-C₃₀-Alkyl ist beispielsweise Methyl, Ethyl, Propyl, i-Propyl, Butyl, i-Butyl, Pentyl, i-Pentyl, Hexyl, i-Hexyl, die isomeren Heptyle, Oktyle, Decyle, Dodecyle, Hexadecyle, Oktadecyle, Eicosyle, Triakontanyle; hiervon sind die C₁-C₁₈-Alkylreste bevorzugt, besonders bevorzugt die C₁-C₈-Alkylreste, ganz besonders bevorzugt die C₁-C₄-Alkylreste, vor allem Methyl und Ethyl.

Geradkettiges oder verzweigtes C₂-C₃₀-Alkenyl ist beispielsweise Vinyl, Propenyl, i-Propenyl, Allyl, die isomeren Butenyle, i-Butenyle, Hexenyle, Oktenyle, Decenyle, Dodecenyle, Hexadecenyle, Eicosenyle, Triakontenyle; bevorzugt sind die C₂-C₄-Alkenyle, vor allem Vinyl.

Halogen ist beispielsweise Fluor, Chlor, Brom, Jod, bevorzugt Fluor, Chlor, Brom, besonders bevorzugt Fluor und Chlor.

Geradkettiges oder verzweigtes C₁-C₈-Alkoxy, C₁-C₈-Alkylamino, Di-(C₁-C₈-alkyl)amino und C₁-C₈-Alkoxycarbonyl enthalten Alkylreste der oben genannten Art; beispielsweise seien genannt: Methoxy, Ethoxy, Methylamino, Ethylamino, Dimethylamino, Diethylamino, Methoxycarbonyl, Ethoxycarbonyl.

Für den Fall, daß zwei ortho-ständige Reste einen gesättigten oder einen einfach oder zweifach ungesättigten Carbocylus bilden, hat dieser zusammen mit dem Benzolring des Arylamins der Formel (II) bzw. (III) 5 oder 6 Ringglieder. Man gelangt damit vom System des Benzols zu dem des Indans, Indens, Tetrahydronaphtalins, Dihydronaphtalins bzw. Naphthalins.

Bevorzugt sind die Substituenten R¹, R², R³, R⁵, R⁶ und R⁷ in den allgemeinen Formeln (I), (II) und (III) gleich oder verschieden und stehen für Wasserstoff, gradkettiges oder verzweigtes C₁-C₁₈ Alkyl, Fluor, Chlor, Methoxy, Trifluormethyl oder Phenyl, wobei jeweils zwei ortho-ständige Substituenten aus R¹, R² und R³ und/oder aus R⁵, R⁶ und R⁷ gemeinsam mit dem zugrundeliegenden Benzolring das System des Indans, Indens, Tetrahydronaphthalins bzw. Napthalins bilden können, während die Substituenten R⁴ und R⁸ für Wasserstoff stehen.

Besonders bevorzugt sind die Substituenten R¹, R², R⁵ und R⁶ in den allgemeinen Formeln (I), (II) und (III) gleich oder verschieden und stehen für Wasserstoff, C₁-C₈-Alkyl, Fluor, Chlor, Methoxy oder Phenyl, wobei ortho-ständige Substituenten R¹ und R² und/oder R⁵ und R⁶ gemeinsam mit dem zugrundeliegenden Benzolring das System des Indans bzw. Naphthalins bilden können, während die Substituenten R³, R⁴, R⁷ und R⁸ für Wasserstoff stehen.

Insbesondere bevorzugt handelt es sich bei den Verbindungen der allgemeinen Formeln (II) und (III) unabhängig voneinander um Anilin, um o- oder p-Toluidin, um p-C₁-C₈-Alkyl-anilin oder um 1-Naphthylamin.

Ausdrücklich genannt sei der Fall, daß es sich bei den Verbindung der allgemeinen Formel (II) um Anilin und bei der Verbindung der allgemeinen Formel (III) um 1-Naphthylamin handelt. Selbstverständlich läßt sich auch das wichtige Diphenylamin erfindungsgemäß erfolgreich herstellen, wenn sowohl (II) als auch (III) Anilin sind. Da jedoch für die Herstellung von Diphenylamin auch andere Verfahren zur Verfügung stehen, hat das hier offenbarte Verfahren besondere Bedeutung zur Herstellung von Diarylaminen der Formel (I) mit Ausnahme des Diphenylamins.

Als Katalysatoren für das erfindungsgemäße Verfahren zur Herstellung von sekundären Diarylaminen lassen sich Katalysatoren einsetzen, die Fluor und gegebenenfalls Bor enthalten, wobei zum Unterschied zu dem in DE-OS 2521293 beschriebenen Verfahren als erfindungsgemäße Katalysatoren Ammoniumfluorid und/oder Ammoniumbifluorid oder aus Ammoniumfluorid und/oder Ammoniumbifluorid und einer Bor enthaltenden Komponente hergestellte katalytisch wirksame Verbindungen eingesetzt werden. Besonders vorteilhaft ist es dabei, daß bei beiden generellen Varianten auf die Handhabung des hochkorrosiven und stark giftigen Fluorwasserstoffs verzichtet werden kann.

### a) Ammoniumfluorid und/oder Ammoniumbifluorid

Für den Fall, daß lediglich Ammoniumfluorid und/oder Ammoniumbifluorid selbst als Katalysatoren verwendet werden, setzt man diese Stoffe in fester Form oder auch in wäßriger Lösung zu. Auch wiedergewonnene Katalysatormengen, die im Zuge der Aufarbeitung von Reaktionsgemischen erhalten werden, lassen sich verwenden. Grundsätzlich ist es auch möglich, Gemische aus wiedergewonnenem und frisch hergestelltem Katalysator zum Einsatz zu bringen.

### b) Fluor und Bor enthaltende Katalysatorsysteme

Erfindungsgemäß einzusetzende Bor enthaltende Verbindungen sind beispielsweise Borsäure H₃BO₃, Boroxid B₂O₃, Borax Na₂B₄O₇, 10H₂O, Ammoniumtetrafluoroborat NH₄(BF₄), bevorzugt Borsäure. Als typische Fluor und Bor enthaltende Katalysatoren für das erfindungsgemäße Verfahren werden im Falle des Einsatzes frischer Katalysatorchargen bevorzugt die Reaktionsgemische verwendet, die bei der Umsetzung von Ammoniumfluorid und/oder Ammoniumbifluorid, Borsäure und dem bzw. den jeweils zur Reaktion gebrachten primären aromatischen Aminen (II) bzw. (III) erhalten werden. Bei den resultierenden katalytisch wirksamen Stoffen handelt es sich um salzartige Produkte der Formel (IV)

[A] [B] (IV),

in der
- A: für die Kationen [H]⁺, [NH₄]⁺, [Aryl-NH₃]⁺ oder [Aryl-NH₂-Aryl]⁺, bevorzugt für die Kationen [H]⁺, [NH₄]⁺ oder [Aryl-NH₃]⁺ und besonders bevorzugt für die Kationen [NH₄]⁺ oder [Aryl-NH₃]⁺ steht und
- B: für Anionen des Typs [B(OH)ₙF₄₋ₙ]⁻, in der n ganzzahlige Werte von 0 bis 4 annehmen kann, und bevorzugt für das Anion [BF₄]⁻ steht.

Aryl ist hierbei der den Formeln (II) bzw. (III) zugrundeliegende Phenylrest oder Naphthylrest. Wichtige Einzelverbindungen der Formel (IV) sind etwa [NH₄]⁺[BF₄]⁻ , [Phenyl-NH₃]+[BF₄]⁻ und [l-Naphthyl-NH₃]+[BF₄]-, die auch als Gemisch vorliegen können. In der Praxis können die innerhalb der erfindungsgemäßen Reaktion wirksamen Katalysatoren, im folgenden vereinfacht "erfindungsgemäßer Bor und Fluor enthaltender Katalysator" genannt, Gemische verschiedener der allgemeinen Formel (IV) entsprechenden Komponenten sein, deren Zusammensetzung sich jedoch nach Maßgabe der stöchiometrischen Verhältnisse sowie der Basizitäten und Dissoziationskonstanten der gegebenenfalls beteiligten kationischen Hauptkomponenten Wasser, Ammoniak und der zur Umsetzung gebrachten aromatischen Amine ergeben und die im allgemeinen auch nicht chemisch gebundene Anteile der Komponenten Wasser, Ammoniak, Anilin und/oder der zum Einsatz gebrachten aromatischen Amine sowie gegebenenfalls noch weitere aromatische Diamine, auch in unprotonierter Form, enthalten. Diese Gemische verschiedener katalytisch wirksamer, solvatisierender oder auch nur solvatisierter Komponenten liegen unter den Reaktionsbedingungen zur erfindungsgemäßen Herstellung der aromatischen Diamine im allgemeinen in homogen gelöster Form vor, wirken demnach also in ihrer Gesamtheit als homogene Katalysatorsysteme.

Die Herstellung von frischen Chargen des erfindungsgemäßen Fluor und gegebenenfalls Bor enthaltenden Katalysators erfolgt im allgemeinen unter Anwendung von dem Fachmann bekannten Verfahren. Grundsätzlich ist es möglich und kann von Vorteil sein, diese Herstellung vorgelagert, in räumlich getrennten, gesonderten Reaktionsgefäßen, vorzunehmen und das so erhaltene Gemisch in das Reaktionsgefäß, in dem die Herstellung des Diarylamins stattfinden soll, einzudosieren. So ist es beispielsweise besonders günstig, zunächst Ammoniumfluorid und/oder Ammoniumbifluorid in reiner Form oder in wäßrigen Lösungen mit Borsäure und aromatischem Amin zur Reaktion zu bringen.

Im allgemeinen verwendet man diejenigen aromatischen Amine für eine vorgelagerte Herstellung des erfindungsgemäßen Bor und Fluor enthaltenden Katalysators, die den zu synthetisierenden Diarylaminen zugrunde liegen. Beispielsweise wird man für die Herstellung von Diphenylamin den dafür eingesetzten erfindungsgemäßen Bor und Fluor enthaltenden Katalysator aus Ammoniumfluorid und/oder Ammoniumbifluorid, Borsäure und Anilin zubereiten. Die für die Herstellung des Fluor enthaltenden Katalysators verwendete Borsäure wird bevorzugt als Feststoff eingesetzt. Grundsätzlich ist es aber auch möglich und kann gegebenenfalls von Vorteil sein, die Borsäure als Schmelze, als Lösung oder als Aufschlämmung, beispielsweise in Wasser und/oder einem aromatischen Amin wie beispielsweise Anilin, zum Einsatz zu bringen.

Zur vorgelagerten Herstellung des erfindungsgemäßen Fluor und Bor enthaltenden Katalysators werden die beteiligten Komponenten im Gewichtsverhältnis Arylamin:Borsäure: Ammoniumfluorid und/oder Ammoniumbifluorid = (100 bis 2000) : (50 bis 250) : (50 bis 250) zum Einsatz gebracht. Bevorzugt bringt man Arylamin, Borsäure und und Ammoniumfluorid und/oder Ammoniumbifluorid im Gewichtsverhältnis von von (300 bis 1000) : (100 bis 150) : (100 bis 150) zum Einsatz. Die Reihenfolge der Dosierung der genannten Komponenten ist grundsätzlich frei wählbar. In einer bevorzugten Ausführungsform wird zunächst Anilin vorgelegt, danach Borsäure hinzugefügt und schließlich Ammoniumfluorid und/oder eindosiert.

Die vorgelagerte Herstellung des erfindungsgemäßen Fluor und Bor enthaltenden Katalysators erfolgt im allgemeinen bei einer Reaktionstemperatur zwischen 0 und 250°C, bevorzugt zwischen 30 und 200°C, besonders bevorzugt zwischen 70 und 150°C.

Bei der vorgelagerten Herstellung des erfindungsgemäßen Fluor und Bor enthaltenden Katalysators kann es zweckmäßig sein zu rühren. Grundsätzlich ist es aber auch möglich, auf zusätzliches Rühren zu verzichten. Im allgemeinen erfolgt die vorgelagerte Herstellung des erfindungsgemäßen Fluor und Bor enthaltenden Katalysators bei Normaldruck. Grundsätzlich ist es aber auch möglich, die Umsetzung bei erniedrigtem oder erhöhtem Druck durchzuführen.

Der solchermaßen hergestellte erfindungsgemäße Fluor und Bor enthaltende Katalysator wird im allgemeinen in flüssiger Form ohne Isolierung oder weitere Aufreinigung in die Reaktion zur Herstellung des angestrebten Diarylamins eingebracht. Grundsätzlich ist es aber auch möglich, den erfindungsgemäßen Fluor und Bor enthaltenden Katalysator etwa unter reduziertem Druck zur Trockne einzudampfen und den dabei anfallenden festen Rückstand als katalytisch wirksames Gemisch in die Reaktion zur Herstellung des angestrebten Diarylamins einzubringen. Gegebenenfalls kann es auch sinnvoll sein, den erfindungsgemäßen Fluor und Bor enthaltenden Katalysator nicht in einer vorgelagerten Umsetzung herzustellen, sondern die Gesamtmenge des zur Synthese des angestrebten Diarylamins benötigten Arylamins sowie die in katalytischen Mengen benötigten Komponenten Ammoniumfluorid und/oder Ammoniumborfluorid und Borsäure in einem einzigen Schritt miteinander zur Reaktion zu bringen. Als erfindungsgemäße Fluor und Bor enthaltende Katalysatoren lassen sich entweder frisch hergestellte Katalysatorchargen oder wiedergewonnene Katalysatormengen, die im Zuge der Aufarbeitung von Reaktionsgemischen erhalten werden, verwenden. Grundsätzlich ist es auch möglich, Gemische aus wiedergewonnenem und frisch hergestelltem Katalysator zum Einsatz zu bringen.

Das erfindungsgemäße Verfahren läuft mit folgenden wesentlichen Schritten ab: Reaktion der primären Arylamine (II) und (III) in Gegenwart des Katalysators; Extraktion des Katalysators; Wäsche der nach der Extraktion verbliebenen organischen Phase; Auftrennung der organischen Phase etwa durch Destillation oder Kristallisation. Zum sparsamen Umgang mit Wasser kann das zur Wäsche eingesetzte und nach Phasentrennung wiedergewonnene Wasser zur Extraktion erneut benutzt werden; ein Teil des nach Phasentrennung erhaltenen Extraktionswassers mit dem Katalysator wird als Katalysatorlösung in die Reaktion zurückgefahren.

Das nach Reaktionsende anfallende flüssige Produktgemisch wird demnach mit Wasser und/oder einer wäßrigen Lösung des gewählten Katalysators versetzt und in einer hierzu geeigneten Apparatur in eine überwiegend wäßrige und eine überwiegend organische flüssige Phase aufgetrennt. Die den größten Teil des organischen Materials enthaltende flüssige Phase, die im wesentlichen das als Reaktionsprodukt entstandene Diarylamin, nicht umgesetztes Ausgangsmaterial sowie kleine Anteile an Nebenprodukten enthält, wird abgetrennt und der weiteren Aufarbeitung zugeführt.

Die den größten Teil des Katalysators enthaltende überwiegend wäßrige Phase wird ebenfalls abgetrennt. Sie kann ausgeschleust werden oder ohne weitere Reinigung oder Aufarbeitung erneut als Katalysator für die Durchführung der erfindungsgemäßen Umsetzung des bzw. der primären aromatischen Amine Verwendung finden.

Die den größten Teil des organischen Materials enthaltende flüssige Phase wird unter Zusatz von Wasser sowie gegebenenfalls eines zusätzlichen anorganischen Hilfsstoffes gewaschen und danach der bevorzugt destillativen Aufarbeitung unterzogen. Bei der abschließenden, bevorzugt destillativen Aufarbeitung des Reaktionsproduktes fällt das erzeugte Diarylamin im allgemeinen in sehr reiner Form an. Überschüssiges primäres Arylamin bzw. überschüssige primäre Arylamine werden dabei im allgemeinen gleichfalls in einer solchen Güte erhalten, daß sie sich problemlos in den Herstellungsprozeß zurückführen lassen. Geringe Mengen Destillationsrückstände werden einer Reststoffverwertung zugeführt oder verworfen.

Die Durchführung des erfindungsgemäßen Verfahrens zur Herstellung von sekundären Diarylaminen kann grundsätzlich in seiner Gesamtheit oder in Teilbereichen auf diskontinuierliche oder auf kontinuierliche Weise erfolgen.

Der Aggregatzustand der als Reaktanden eingebrachten Arylamine ist grundsätzlich belanglos. In einer bevorzugten Ausführungsform werden die Edukte in flüssiger Form eingebracht. Es kann aber auch von Vorteil sein, die Edukte als Feststoffe oder auch in verdampfter Form in das Reaktionsgemisch einzubringen.

Bei der gezielten Herstellung unsymmetrischer sekundärer Diarylamine kann es besonders vorteilhaft sein, eine Reaktionskomponente oder ein Gemisch der einen Reaktionskomponente mit einem Teil der anderen gemeinsam mit dem gewählten Katalysatorsystem vorzulegen und den restlichen Teil der anderen Reaktionskomponente im Verlaufe der fortschreitenden Umsetzung in das Reaktionsgefäß einzudosieren.

Der entsprechend dem erfindungsgemäßen Verfahren in die Reaktion zur Herstellung der sekundären Diarylamine eingebrachte Katalysator bzw. das Katalysatorgemisch wird in einer solchen Menge eingesetzt, daß das molare Verhältnis zwischen der katalytisch wirksamen Komponente im Katalysator bzw. dem Katalysatorgemisch und der gesamten Stoffmenge des bzw. der in die Reaktion eingesetzten primären Arylamine zwischen 1 : 10 und 1 : 100.000, bevorzugt zwischen 1 : 25 und 1 : 10.000 und besonders bevorzugt zwischen 1 : 50 und 1 : 1.000 liegt. Der erfindungsgemäße Katalysator bzw. das Katalysatorgemisch wird im allgemeinen in flüssiger oder fester Form, als Reinstoff, Lösung oder Anschlämmung in das Reaktionsgemisch eingebracht. In einer besonderen Ausführungsform wird der erfindungsgemäße Katalysator bzw. das Katalysatorgemisch nach Eindampfen katalysatorhaltiger Lösungen, wie sie etwa bei der Herstellung frischer Katalysatorchargen oder bei der Wiedergewinnung von Katalysator im Zuge der weiteren Aufarbeitung ausreagierter Reaktionsgemische anfallen, in fester Form als katalytisch wirksamer Feststoff oder katalytisch wirksames Feststoffgemisch in die erfindungsgemäße Reaktion zur Herstellung der sekundären Diarylamine zurückgeführt.

Die erfindungsgemäße Reaktion zur Herstellung von sekundären Diarylaminen wird im Druckbereich zwischen Normaldruck und 30 bar, bevorzugt zwischen 1 und 20 bar, besonders bevorzugt zwischen 1 und 15 bar, durchgeführt.

Das erfindungsgemäße Verfahren wird im Temperaturbereich zwischen 100 und 400°C, bevorzugt zwischen 150 und 350°C, besonders bevorzugt zwischen 180 und 300°C, durchgeführt. Zur Gewährleistung des gewählten Temperaturniveaus wird dem Reaktionsgefäß durch außen- und/oder innenliegende Heizaggregate die erforderliche Wärmeenergie zugeführt.

Das Reaktionsgut wird im Verlaufe des erfindungsgemäßen Verfahrens durchmischt. Dies kann beispielsweise durch Rühraggregate, durch außen- oder innenliegende Umpumpvorrichtungen oder auf eine andere geeignete Weise geschehen. Grundsätzlich ist es aber auch möglich, im Verlaufe der Reaktion auf jegliche aktive Durchmischung des Reaktionsgutes zu verzichten, da das im Zuge der Umsetzung der Reaktanden freiwerdende Ammoniakgas sowie siedende Reaktionskomponenten zu einer Durchmischung führen.

Das im Zuge der Umsetzung der Reaktanden entweichende Ammoniakgas wird abgetrennt und einer weiteren Verwertung oder einer geregelten Entsorgung zugeführt. Die Abtrennung des im Zuge der Umsetzung der Reaktanden entweichenden Ammoniakgases erfolgt im allgemeinen mit Hilfe dazu dem Fachmann bekannter geeigneter Kondensatoren und Flüssigkeitsabscheider, deren Betriebsbedingungen so gewählt werden, daß sie den Durchtritt des Ammoniakgases erlauben, die erheblich höher siedenden Reaktanden sowie die Reaktionsprodukte jedoch im Reaktionsbereich zurückhalten.

Neben den genannten Reaktionsbedingungen sowie den apparativen Parametern ist die zeitliche Dauer der erfindungsgemäßen Reaktion zur Herstellung von sekundären Diarylaminen maßgeblich für den erzielten Umsetzungsgrad der eingesetzten Reaktanden. Da das erfindungsgemäße Verfahren zur Herstellung sekundärer Diarylamine die Rückführung nicht umgesetzter primärer Arylamine ermöglicht, ist die Reaktionszeit in weiten Grenzen variierbar und kann unter prozeßökonomischen Gesichtspunkten beispielsweise so gewählt werden, daß die Raum-Zeit-Ausbeuten des Reaktionsteils des erfindungsgemäßen Verfahrens möglichst hoch und der trenntechnische Aufwand zur Rückführung nicht umgesetzten Ausgangsmaterials möglichst klein ist. Im allgemeinen werden mehr als 30 %, bevorzugt mehr als 35 %, besonders bevorzugt mehr als 45 % des bei diskontinuierlicher Verfahrensweise in einen diskreten Reaktionsansatz bzw. bei kontinuierlicher Verfahrensweise des bzw. der in die Reaktionszone eingebrachten primären Arylamine umgesetzt.

Das nach Beendigung der erfindungsgemäßen Reaktion zur Herstellung von sekundären Diarylaminen anfallende Produktgemisch wird, bevorzugt nach Abkühlen auf eine Temperatur unterhalb von 100°C, zunächst mit Wasser und/oder wasserhaltiger Lösung wiedergewonnenen Katalysatorgemisches aus vorangegangenen Aufarbeitungen versetzt und vermischt. Das dabei anfallende zweiphasige Gemisch wird einer Phasentrennung zugeführt. Die Menge des in diesen Extraktionsprozeß eingesetzten Wassers und/oder der wasserhaltigen Lösung aus vorangegangenen Aufarbeitungen wiedergewonnenen Katalysatorgemisches wird vorzugsweise so gewählt, daß der nach der Phasentrennung schließlich anfallende wäßrige Extrakt ca. 1 - 35 % nicht verdampfbare katalytisch wirksame Komponenten enthält. Die Temperatur des zweiphasigen Gemisches wird im Verlauf der Extraktion und der Phasentrennung im allgemeinen im Bereich zwischen 40 und 100°C gehalten. Die nach der Phasentrennung erhaltene organische Phase wird vorzugsweise einer weiteren Extraktion unterworfen. Grundsätzlich ist es aber auch möglich, sie ohne weitere Behandlung aufzudestillieren. Eine zweite Extraktion zur Wäsche der organischen Phase erfolgt mit Wasser, dem gegebenenfalls zwischen 0 und 50 %, bevorzugt zwischen 5 und 30 %, besonders bevorzugt zwischen 10 und 20 % eines oder mehrerer Hilfsstoffe, bevorzugt eines oder mehrerer salzartiger anorganischer Hilfsstoffe, besonders bevorzugt eines oder mehrerer Stoffe aus der Gruppe der Alkali- und Erdalkalimetallhalogenide, der Alkalimetallsulfate, der Alkalimetallcarbonate und der Alkali- und Erdalkalimetallhydrogencarbonate zugesetzt wurde. Die Temperatur des zweiphasigen Gemisches wird im Verlauf der Extraktion und der Phasentrennung im allgemeinen im Bereich zwischen 20 und 90°C gehalten.

Das bei der zweiten Extraktion anfallende zweiphasige Gemisch wird einer Phasentrennung zugeführt. Die nach der Phasentrennung erhaltene wäßrige Phase wird zum Teil für weitere Extraktionen wiederverwendet, zum Teil ausgeschleust und einer Verwertung oder einer geregelten Entsorgung zugeführt. Die nach der Phasentrennung erhaltene organische Phase wird bevorzugt der destillativen Auftrennung zugeführt. Beispielsweise mit Hilfe konventioneller, dem Fachmann bekannter Destillationsapparaturen und Destillationstechniken wird diese destillative Auftrennung vorzugsweise so durchgeführt, daß in dem rohen Destillationsgemisch vorhandenes nicht umgesetztes primäres Arylamin nahezu vollständig wiedergewonnen und in spätere Reaktionen zur Herstellung des sekundären Diarylamins wiedereingesetzt werden kann.

### Beispiele

### Vergleichsversuch 1

Zu einem Gemisch aus 8,2 kg (88,05 mol) Anilin und 7,2 kg (50,28 mol) 1-Naphthylamin gab man 0,3 kg (0,6 mol) einer aus wäßriger Flußsäure, NH₃-Wasser und Borsäure bereiteten 21 %igen Lösung von Ammoniumtetrafluoroborat in Wasser. Man erhitzte etwa 10 Stunden lang unter einem Druck von 20 mbar auf eine Temperatur von ca. 100°C, wobei ca. 1,0 kg eines Gemisches aus Anilin und Wasser abdestillierten. Man fügte dem Reaktionsgemisch weitere 1,0 kg (10,74 mol) Anilin hinzu und erhitzte nun während 20 Stunden unter Normaldruck auf Temperaturen zwischen 200 und 215°C. Siedendes Anilin wurde durch einen aufgesetzten Kondensator am Entweichen gehindert, während das entstehende Ammoniakgas am Kopf des Kondensators abgeleitet wurde. Nach beendeter Umsetzung entnahm man dem Reaktionsgemisch eine Probe, die gaschromatographisch analysiert wurde.

| | | |
|---|---|---|
| Ergebnis: | Anilin | 29,1 % |
| | 1 -Naphthylamin | 6,5 % |
| | N-Phenyl-1-naphthylamin | 60,0 % |
| | Diphenylamin | <0,1 % |

### Vergleichsversuch 2

### Herstellung des Katalysatorgemisches

In einem Edelstahlkessel wurden 5,0 kg (53,69 mol) Anilin vorgelegt und bei Raumtemperatur während ca. 15 Minuten unter Rühren mit 1,20 kg (19,41 mol) Borsäure versetzt. Im Verlauf von ca. 120 Minuten ließ man unter Rühren 2,00 kg (72,96 mol) einer 73%igen wäßrigen Flußsäurelösung zulaufen, während die Temperatur des Reaktionsgemisches mittels äußerer Kühlung innerhalb eines Bereiches zwischen 20 und 60°C gehalten wurde.

### Durchführung der Reaktion

Wie in Vergleichsversuch 1, jedoch unter Verwendung von 0,3 kg des aus Anilin, Borsäure und wäßriger Flußsäure frisch bereiteten Katalysatorgemisches anstelle der wäßrigen Ammoniumtetrafluoroborat-Lösung. Nach Abdestillieren des Wasser-Anilin-Azeotrops ergänzte man, wie oben beschriebenen, den Gewichtsverlust durch Zusatz von frischem Anilin und erhitzte 20 Stunden lang unter Rückfluß, wobei die Sumpftemperatur langsam auf ca. 215°C anstieg. Nach beendeter Umsetzung entnahm man dem Reaktionsgemisch eine Probe, die gaschromatographisch analysiert wurde.

| | | |
|---|---|---|
| Ergebnis: | Anilin | 28,5 % |
| | 1-Naphthylamin | 6,1 % |
| | N-Phenyl-1-naphthylamin | 61,5 % |
| | Diphenylamin | <0,1 % |

### Beispiel 1

Ein Gemisch aus 429,43 g (4611 mmol) Anilin, 363,77 g (2540 mmol) 1-Naphthylamin und 11,06 g (299 mmol) Ammoniumfluorid wurde unter Rühren und Rückfluß erhitzt. Dabei stieg die Sumpftemperatur des Reaktionsgemisches kontinuierlich bis auf einen Wert von 217°C. Nach 12 und nach 20 h Reaktionszeit wurde dem Reaktionsgemisch Proben entnommen, die gaschromatographtisch analysiert werden.

| | | | |
|---|---|---|---|
| Ergebnis: | | 12 h | 20 h |
| | Anilin | 27,9 % | 25,0 % |
| | 1-Naphthylamin | 12,8 % | 6,9 % |
| | N-Phenyl-l-naphthylamin | 54,7 % | 63,0 % |
| | Diphenylamin | <0,1 % | <0,1 % |

### Beispiel 2

Durchführung wie in Beispiel 1, jedoch unter Verwendung von 4,19 g (74 mmol) Ammoniumbifluorid als Katalysator. Die Sumpftemperatur des Reaktionsgemisches stieg kontinuierlich bis auf einen Wert von 215°C. Nach 12 h Reaktionszeit wurde dem Reaktionsgemisch eine Probe entnommen, die gaschromatographtisch analysiert wurde.

| | | |
|---|---|---|
| Ergebnis: | Anilin | 39,8 % |
| | 1-Naphthylamin | 25,7 % |
| | N-Phenyl-1-naphthylamin | 31,5 % |
| | Diphenylamin | <0,1 % |

### Beispiel 3

Durchführung wie in Beispiel 1, jedoch unter Verwendung von 11,06 g (299 mmol) Ammoniumbifluorid und 2,66 g (43 mmol) Borsäure als Katalysator. Die Sumpftemperatur des Reaktionsgemisches stieg kontinuierlich bis auf einen Wert von 212°C. Nach 12 h Reaktionszeit wurde dem Reaktionsgemisch eine Probe entnommen, die gaschromatographtisch analysiert wurde.

| | | |
|---|---|---|
| Ergebnis: | Anilin | 27,9 % |
| | 1 -Naphthylamin | 5,5 % |
| | N-Phenyl-1-naphthylamin | 62,1 % |
| | Diphenylamin | <0,1 % |

### Beispiel 4

Durchführung wie in Beispiel 1, jedoch unter Verwendung von 4,19 g (74 mmol) Ammoniumbifluorid und 2,61 g (42 mmol) Borsäure als Katalysator. Die Sumpftemperatur des Reaktionsgemisches steigt kontinuierlich bis auf einen Wert von 215°C.

Nach 12 h Reaktionszeit wurde dem Reaktionsgemisch eine Probe entnommen, die gaschromatographtisch analysiert wurde.

| | | |
|---|---|---|
| Ergebnis: | Anilin | 28,6 % |
| | 1-Naphthylamin | 3,6 % |
| | N-Phenyl-1-naphthylamin | 63,3 % |
| | Diphenylamin | <0,1 % |

## Patentansprüche

1. Verfahren zur Herstellung von sekundären Diarylaminen der allgemeinen Formel (I) 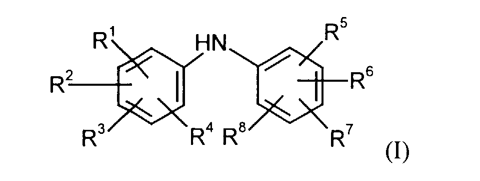 durch Umsetzung von primären Arylaminen der Formel (II) 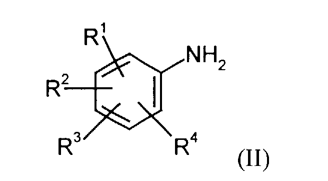 mit primären aromatischen Aminen der Formel (III) 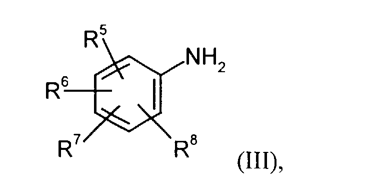 wobei
R¹, R², R³, R⁴, R⁵, R⁶, R⁷ und R⁸ gleich oder verschieden sind und für Wasserstoff, geradkettiges oder verzweigtes C₁-C₃₀-Alkyl, C₂-C₃₀-Alkenyl, Halogen, C₁-C₈-Alkoxy, Trifluormethyl, C₁-C₈-Alkylamino, Di(C₁-C₈-alkyl)amino, Carboxyl, C₁-C₈-Alkoxycarbonyl oder Phenyl stehen, wobei jeweils zwei ortho-ständige Substituenten aus R¹, R², R³ und R⁴ und/oder aus R⁵, R⁶, R⁷ und R⁸ einen gesättigten oder einen einfach oder zweifach ungesättigten 5- oder 6-gliedrigen Carbocyclus bilden können, dadurch gekennzeichnet, daß die Reaktion diskontinuierlich oder kontinuierlich in Gegenwart eines Fluor und gegebenenfalls Bor enthaltenden Katalysators aus der Gruppe von gegebenenfalls substituiertem Ammoniumfluorid, Ammoniumbifluorid und Katalysatoren, die aus gegebenenfalls substituierem Ammoniumfluorid und/oder Ammoniumfluorid und einer Bor enthaltenden Verbindung hergestellt werden, bei 100-400°C in kondensierter Phase durchgeführt wird, wobei bei Mitverwendung eines Bor enthaltenden Katalysators die Herstellung von N-Phenyl-1-naphthylamin ausgenommen ist,

2. Verfahren nach Anspruch 1, dadurch gekennzeichnet, daß die Substituenten R¹, R², R³, R⁵, R⁶ und R⁷ gleich oder verschieden sind und für Wasserstoff, geradekettiges oder verzweigtes C₁-C₁₈-Alkyl, Fluor, Chlor, Brom, Methoxy, Trifluormethyl oder Phenyl stehen, wobei jeweils zwei ortho-ständige Substituenten aus R¹, R² und R³ und/oder aus R⁵, R⁶ und R⁷ gemeinsam mit dem zugrundeliegenden Benzolring das System des Indans, Indens, Tetrahydronaphthalins bzw. Naphthalins bilden können, während die Substituenten R⁴ und R⁸ für Wasserstoff stehen, daß bevorzugt die Substituenten R¹, R², R⁵ und R⁶ in den allgemeinen Formeln (I), (II) und (II) gleich oder verschieden sind und für Wasserstoff, C₁₋₈-Alkyl, Fluor, Chlor, Methoxy oder Phenyl stehen, wobei ortho-ständige Substituenten R¹ und R² und/oder R⁵ und R⁶ gemeinsam mit dem zugrundeliegenden Benzolring das System des Indans bzw. Naphthalins bilden können, während die Substituenten R³, R⁴, R⁷ und R⁸ für Wasserstoff stehen.

3. Verfahren nach Anspruch 2, dadurch gekennzeichnet, daß es sich bei den Verbindungen der Formeln (II) und (III) unabhängig voneinander um Anilin, um o- oder m-Toluidin, um p-(C₁-C₈)-Alkyl-anilin oder um 1-Naphthylamin handelt, daß es sich bevorzugt bei den Verbindungen der Formel (II) um Anilin und der Formel (III) unabhängig voneinander um Anilin oder 1-Naphtylamin handelt.

4. Verfahren nach Ansprüchen 1 bis 3, dadurch gekennzeichnet, daß man die Reaktion im Druckbereich von 1-30 bar, bevorzugt von 1-20 bar, besonders bevorzugt von 1-15 bar, durchführt.

5. Verfahren nach Ansprüchen 1 bis 4, dadurch gekennzeichnet, daß man die Reaktion im Temperaturbereich von 150 bis 350°C, bevorzugt von 180 bis 300°C, durchführt.

6. Verfahren nach Ansprüchen 1 bis 5, dadurch gekennzeichent, daß man die Reaktion in Gegenwart von Ammoniumfluorid und/oder Ammoniumbifluorid durchführt.

7. Verfahren nach Ansprüchen 1 bis 5, dadurch gekennzeichnet, daß man die Reaktion in Gegenwart eines aus Ammoniumfluorid und/oder Ammoniumbifluorid, Borsäure und einem oder zweier primärer Arylamine bereiteten, Fluor und Bor enthaltenden Katalysators oder Katalysatorgemisches durchführt.

8. Verfahren nach Anspruch 7, dadurch gekennzeichnet, daß es sich bei den katalytisch wirksamen Komponenten des Katalysatorgemisches um salzartige Verbindungen der allgemeinen Formel
[A] [B] (IV)
handelt, in der
A für die Kationen [H]⁺, [NH₄]⁺, [Aryl-NH₃]⁺ oder [Aryl-NH₂-Aryl]⁺, bevorzugt für die Kationen [H]⁺, [NH₄]⁺ oder [Aryl-NH₃]⁺ und besonders bevorzugt für die Kationen [NH₄]⁺ oder [Aryl-NH₃]⁺ steht und
B für Anionen des Typs [B(OH]ₙF₄₋ₙ]⁻, in der n ganzzahlige Werte zwischen einschließlich 0 und einschließlich 4 annehmen kann, und bevorzugt für das Anion [BF₄]⁺ steht,
wobei Aryl der den Formeln (II) und (III) zugrundeliegende Phenylrest oder Naphthylrest ist.

9. Verfahren nach Anspruch 8, dadurch gekennzeichnet, daß es sich bei den katalytisch wirksamen Komponenten des Katalysatorgemisches um die Verbindungen
[NH₄]⁺[BF₄]⁻, [Phenyl-NH₃]⁺[BF₄]⁻ und/oder [1-Naphthyl-NH₃]⁺[BF₄]⁻
handelt.

10. Verfahren nach Ansprüchen 1 bis 9, dadurch gekennzeichnet, daß nach Beendigung der Reaktion eine Extraktion des erhaltenen Produktgemisches mit Wasser oder wasserhaltigen Lösungen zur Gewinnung der katalytisch wirksamen Komponenten durchgeführt wird.
